# EUROPÄISCHE PATENTSCHRIFT

(11) **EP 2 026 229 B1**
(45) Veröffentlichungstag und Bekanntmachung des Hinweises auf die Patenterteilung: **12.09.2018**
(21) Anmeldenummer: 08075269.4
(22) Anmeldetag: 31.03.2008
(51) Int. Cl.: G06F 19/00, H04L 29/06, H04L 29/08, A61N 1/372

(54) **Anordnung und Verfahren zur Verwaltung einer Datenübertragungsschicht für ein persönliches medizinisches Gerät**
Assembly and method for managing a data transfer layer for a personal medicinal device
Agencement et procédé de gestion d'une couche de transmission des données pour un appareil médical personnel

(30) Priorität: 28.07.2007 DE 102007035533
(43) Veröffentlichungstag der Anmeldung: 18.02.2009
(73) Patentinhaber: BIOTRONIK SE & Co. KG, 12359 Berlin (DE)
(72) Erfinder: Dörr, Thomas, 12437 Berlin (DE); Reinke, Joachim, 10439 Berlin (DE)
(74) Vertreter: Keck, Hans-Georg

(56) Entgegenhaltungen:
- DE-A1- 4 131 133
- US-A1- 2001 031 997
- US-A1- 2005 203 582
- US-A1- 2005 204 134
- US-B1- 6 363 282

## Beschreibung

Die vorliegende Erfindung betrifft eine Anordnung zur Verwaltung einer Datenübertragungsschicht für ein programmierbares persönliches medizinisches Gerät, insbesondere eines implantierbaren medizinischen Gerätes wie einem Herzschrittmacher, Defibrillator oder dergleichen sowie eine Verwaltungseineinheit für eine solche Datenübertragungsschicht und ein Verfahren zur Verwaltung einer solchen Datenübertragungsschicht.

Es ist bekannt, Daten zur Programmierung oder Verwaltung von programmierbaren persönlichen medizinischen Geräten wie beispielsweise einem Herzschrittmacher über Datenverbindungen wie ein leitungsgestütztes Telefonnetz, das Internet, ein funkgestütztes Telefonnetz oder ähnliches zwischen einem (zentralen) Dienstleister und dem Herzschrittmacher auszutauschen. Die Datenverbindungen stellen hierbei in der Regel keine sicheren und bedingungslos vertrauenswürdigen Verbindungen dar.

Beim Betrieb eines Herzschrittmachers oder Defibrillators fallen Daten bezüglich des medizinischen Gerätes sowie bezüglich seines Betriebs an. Diese Daten ergeben sich einerseits aus dem Betriebszustand des persönlichen Gerätes selbst und andererseits aus Daten, die durch das persönliche Gerät detektiert werden. Solche Daten sind insbesondere für eine optimale Nachsorge von Bedeutung. Solche Nachsorgedaten, die von einem Implantat aufgenommen wurden, können über die oben erwähnten Verbindungen an den Dienstleister übermittelt werden.

In der Gegenrichtung, also vom Dienstleister zum Implantat, können vom behandelnden Arzt Aktualisierungen der Betriebsparameter oder des Betriebsprogramms des Implantats veranlasst werden.

In beiden Fällen, einerseits aus Gründen der Sicherung der Privatsphäre und der Vertraulichkeit der Patientendaten und andererseits aus Gründen der Betriebssicherheit und Vermeidung von Manipulationen an den Einstellungen des Implantats, werden bei der Datenübertragung vorbestimmte Protokolle oder Module verwendet, wobei insbesondere Ver- bzw. Entschlüsselungs- und Authentifizierungs-Algorithmen eingesetzt werden.

In US 6,442,432 B2 ist die Verschlüsselung einer Datenübertragung zwischen einem medizinischen Implantat und einem zentralen Dienstleister per PGP erwähnt.

Das Dokument US 2005/0204134 A1 offenbart die sichere Authentifizierung einer Datenübertragung mittels kryptographischer Schlüssel.

Auch wenn ein Datenaustausch nicht über möglicherweise unsichere Datenleitungen sondern in einer sicheren Umgebung wie einer Klinik erfolgt, kann auch durch Verwendung entsprechender Protokolle eine Erhöhung der Sicherheit erreicht werden.

Erfahrungsgemäß kommt es dazu, dass zunächst als sicher und ausreichend eingestufte Algorithmen oder Module zu einem späteren Zeitpunkt als doch unsicher oder zumindest nur eingeschränkt sicher eingestuft werden. In einem solchen Fall besteht das Problem, dass die Protokolle, Algorithmen oder Module sowohl an der verschlüsselnden als auch an der entschlüsselnden Stelle bzw. auf beiden Seiten einer Authentifizierung einfach austauschbar sein müssen, ohne dass der Rest der Datenübertragung oder die Nutzdatenlast dafür geändert werden muss.

Die Begriffe "Algorithmus", "Modul" und "Protokoll" sind im Kontext der vorliegenden Erfindung als im Grundsatz synonym zu verstehen und werden im Folgenden austauschbar verwendet.

Eine Aufgabe der vorliegenden Erfindung ist es somit, eine Verwaltung der Datenübertragungsschicht, mit der beispielsweise ein aus der Ferne programmierbares medizinisches Gerät wie ein Implantat (Schrittmacher oder ICD) neue Programmierungen erhält und Daten übermittelt, zu leisten und dabei gleichzeitig für eine ausreichende Sicherheit der Datenübertragungsschicht als auch für eine zuverlässige und ausfallsichere Kommunikationsmöglichkeit mit dem persönlichen Gerät zu sorgen.

Erfindungsgemäß wird diese Aufgabe durch eine Anordnung zur Verwaltung einer Datenübertragungsschicht für ein implantierbares programmierbares persönliches medizinisches Gerät mit den Merkmalen nach Anspruch 1 gelöst.

Die Aufgabe wird auch gelöst durch eine Verwaltungseinheit zur Verwaltung einer Datenübertragungsschicht für ein implantierbares programmierbares persönliches medizinisches Gerät mit den Merkmalen nach Anspruch 16.

Weiterhin wird die Aufgabe gelöst durch ein Verfahren zur Verwaltung einer Datenübertragungsschicht für ein implantierbares programmierbares persönliches medizinisches Gerät nach Anspruch 17.

Die Erfindung beruht auf der Einsicht, dass ein Datenübertragungsprotokoll, dass die Datenübertragungsschicht in einer Softwarearchitektur darstellt, separat von der restlichen in beispielsweise einem Implantat laufenden Software ausgetauscht werden kann, wobei lediglich das Datenübertragungsprotokoll, nicht aber notwendigerweise auch der restliche Betrieb, eine Änderung erfährt. Um diese Änderung, die an dem Datenübertragungsprotokoll, also der Wirkungsweise der Datenübertragungsschnittstelle, des persönlichen Gerätes vorgenommen wird, bei einer weiteren Kommunikation mit dem persönlichen Gerät berücksichtigen zu können, ist erfindungsgemäß vorgesehen, dass eine Abfrage des Status der Datenübertragungsschnittstelle bzw. einer Kennzeichnung des aktuellen Datenübertragungsprotokolls, das von der Datenübertragungsschnittstelle verwendet wird, erfolgt, wobei die weitere Kommunikation unter Einbeziehung des Ergebnisses dieser Abfrage durchgeführt wird. Die erfindungsgemäße Verwaltungseinheit ist dazu ausgebildet, anhand der Rückmeldung auf die Frage, welches Datenübertragungsprotokoll derzeit im persönlichen Gerät eingesetzt, ein passendes Datenübertragungsprotokoll aus den in der Speichereinheit vorgehaltenen Datenübertragungsprotokollen auszuwählen und für einen Datenaustausch mit dem persönlichen Gerät nutzbar zu machen.

Zudem erlaubt die Erfindung, ein in einem persönlich Geräten eingesetztes Datenübertragungsprotokoll durch ein anderes Datenübertragungsprotokoll zu ersetzen oder zu ergänzen, so dass Änderungen an den für die Mehrzahl von persönlichen Geräten verwendeten Datenübertragungsprotokollen zentral von der Verwaltungseinheit gesteuert und veranlasst werden können. Auf Basis der Prüfung, welches Datenübertragungsprotokoll aktuell in einem persönlichen Gerät kann die Verwaltungseinheit ein Datenübertragungsprotokoll aus den im Speicher vorgehaltenen Datenübertragungsprotokollen auswählen und an das persönliche Gerät übermitteln (lassen), so dass beispielsweise gezielt ein bestimmtes, für nicht mehr ausreichend sicher angesehenes Datenübertragungsprotokoll durch ein anderes ersetzt oder ergänzt werden kann.

Die Steuereinheit des persönlichen Gerätes ist dazu eingerichtet, über die Datenübertragungsschnittstelle Daten entgegennehmen zu können, wobei diese Daten von der Datenübertragungsschnittstelle beispielsweise gemäß dem aktuellen Datenübertragungsprotokoll empfangen wurden. Auf Grundlage dieser empfangenen Daten ist die Steuereinheit dazu ausgelegt, eine Änderung an dem Datenübertragungsprotokoll der Datenübertragungsschnittstelle vorzunehmen. Eine solche Änderung kann beispielsweise eine Anpassung einzelner Parameter oder auch der komplette Austausch des gesamten Datenübertragungsprotokolls sein.

Damit unterschiedliche Datenübertragungsprotokolle bei der Verwaltung der Datenübertragungsschicht voneinander unterschieden werden können, ist diesen Datenübertragungsprotokollen jeweils ein Kennzeichen eigen.

In einer Ausführungsform der Erfindung ist wenigstens ein persönliches Gerät der Mehrzahl von persönlichen Geräten ein aktives medizinisches Implantat. Insbesondere bei einem aktiven medizinischen Implantat bestehen besondere Sicherheitsanforderungen an die Kommunikationssicherheit, die mit der vorliegenden Erfindung erfüllt werden.

In einer Ausführungsform der Erfindung ist das persönliche Gerät ein implantierbarer Herzschrittmacher oder Defibrillator-Kardioverter. Gerade beim Betrieb eines Herzschrittmachers oder einen Defibrillator-Kardioverters ist eine sichere Kommunikation von beispielsweise Programmierdaten oder detektierten Herzdaten gemäß der Erfindung von Vorteil.

Ver- und Entschlüsselungsprotokolle sowie Authentifizierungsprotokolle sind Datenübertragungsprotokolle und bilden die Mehrzahl von Datenübertragungsprotokollen. Für eine sichere und zuverlässige Datenübertragung sind insbesondere Ver- bzw. Entschlüsselungsalgorithmen sowie Module zur Verifizierung einer Kommunikationsgegenstelle oder - zwischenstation von Bedeutung. Des Weiteren können die Datenübertragungsprotokolle auch andere Aspekte der Datenübertragung betreffen, wie beispielsweise eine Komprimierung von Daten oder Einstellungen zu Rückmeldungen oder Quittierungen hinsichtlich empfangener Daten.

In einer erfindungsgemäßen Ausführungsform weist die Mehrzahl von Datenübertragungsprotokollen erste Ver- und Entschlüsselungsprotokolle sowie Authentifizierungsprotokolle zur Verwendung in der Datenübertragungsschnittstelle eines persönlichen Gerätes und zweite Ver- und Entschlüsselungsprotokolle sowie Authentifizierungsprotokolle zur Verwendung beim Zurverfügungstellen durch die Verwaltungseinheit auf. Bei einer asymmetrischen Struktur der Datenübertragungsprotokolle unterscheidet sich ein Verschlüsselungsmodul oder -protokoll vom dazugehörigen Entschlüsselungsprotokoll oder - modul. Entsprechendes gilt für eine Authentifizierung oder Verifizierung des jeweiligen Kommunikationsteilnehmers. Ein Datenübertragungsprotokoll für das Zurverfügungstellen kann so ausgestaltet sein, dass es mit mehreren Datenübertragungsprotokollen, die bei unterschiedlichen persönlichen Geräten Einsatz finden, kompatibel ist, also erfolgreich eine Datenübertragung durchführen kann, während die entsprechenden Datenübertragungsprotokolle jeweils nur mit diesem einen Datenübertragungsprotokoll und nicht mit anderen Protokolle eine erfolgreiche Kommunikation aufbauen bzw. durchführen können. Damit besteht für das zur Verfügung zu stellenden bzw. bereitzuhaltende Datenübertragungsprotokoll eine größere Flexibilität, ohne dass dabei eine Verringerung der Sicherheit aufträte.

Bei einer vorteilhaften Ausführungsform der Erfindung ist die Datenübertragungsschnittstelle mit wenigstens zwei Datenübertragungsprotokollen versehen, insbesondere mit einem ersten Protokoll zur Verschlüsselung und einem zweiten Protokoll zur Entschlüsselung einer Datenübertragung. Es besteht damit die Möglichkeit, für die unterschiedlichen Kommunikationsrichtungen unterschiedliche Protokolle vorzusehen, wobei beispielsweise auch auf die unterschiedlichen Rechenkapazitäten von Implantat als persönlichem Gerät und externer Vorrichtung zum Programmieren des Implantats Rücksicht genommen werden kann. Ferner wird für eine bei persönlichen Gerät eingehende Datenübertragung, beispielsweise eine Programmierung, ein anderes Datenübertragungsprotokoll als für eine ausgehende Datenübertragung, beispielsweise Nachsorgedaten, vorgesehen, wobei hierbei ein jeweilig angepasster Kompromiss zwischen Geschwindigkeit und Sicherheit eingestellt werden kann.

Gemäß einer weiteren Ausführungsform der Erfindung umfasst das Kennzeichen eine Versionsnummer, ein Erstellungsdatum, ein Identifikationszeichen und/oder eine Klassifikation des Datenübertragungsprotokolls. Eine Mehrzahl von möglichen Kennzeichen kann dazu dienen, ein Datenübertragungsprotokoll bzw. eine Version eines Datenübertragungsprotokolls von einem anderen bzw. einer anderen Version unterscheidbar zu machen. Typischerweise werden bei der Erstellung von aufeinander aufbauenden bzw. weiterentwickelten Datenübertragungsprotokollen Versionsnummern vergeben, die einen Rückschluss auf die Aktualität des Protokolls geben können. Ähnliches gilt für die Vergabe eines Datums, an dem das Protokoll freigegeben, erstellt oder zum ersten Mal eingesetzt wurde. Ein Identifikationszeichen kann eine Kennung sein, die unabhängig von der Version oder Klasse des Protokolls für eine bestimmte Instanz des Protokolls vergeben wurde, wobei dieses Identifikationszeichen somit speziell für das in dem persönlichen Gerät eingesetzte Protokoll wäre und sich von dem vieler oder aller anderen Protokolle unterscheiden würde. Das Identifikationszeichen kann allerdings auch eine aus dem Protokoll selbst erzeugte Kennung sein, beispielsweise ein Hash-Wert, der auf dem Protokollcode erzeugt wurde. Bei einem Nachhalten und regelmäßigen Überprüfen einer solchen Kennung könnte zudem eine Überprüfung der Integrität des Protokolls geleistet werden.

Gemäß einer weiteren erfindungsgemäßen Ausführungsform ist die Abfrageeinheit zur Abfrage des Kennzeichens aus einer Datenbank der Anordnung auf Basis der Identifikation des persönlichen Gerätes und/oder zur Abfrage des Kennzeichens durch Abfragen des persönlichen Gerätes ausgebildet, wobei das persönliche Gerät zur Beantwortung einer Abfrage der Abfrageeinheit durch Übermittlung des Kennzeichens ausgebildet ist. Ein Nachhalten einer Zuordnung von Datenübertragungsprotokoll und persönlichem Gerät über das Kennzeichen und die Identifikation in einer Datenbank kann das passende Datenübertragungsprotokoll für eine Kommunikation mit einem bestimmten persönlichen Gerät (insbesondere eine Sendung an dieses Gerät) unter Verwendung des richtigen Protokolls erfolgen, ohne das zunächst eine Überprüfung oder Kommunikation mit dem persönlichen Gerät selbst erfolgen müsste. Dies ist insbesondere dann vorteilhaft, wenn es sich um eine asynchrone Übermittlung von Daten an das persönliche Gerät handelt. Wird beispielsweise ein Programmpaket zur Übermittlung an ein persönliches Gerät vorbereitet, so kann dieses Programmpaket nach der Datenbankabfrage direkt gemäß dem Datenübertragungsprotokoll, beispielsweise gemäß der passenden Verschlüsselung, vorbereitet und zum dem persönlichen Gerät gesendet werden. Die Absendung und der Empfang können hierbei zeitlich getrennt stattfinden, wobei das beispielsweise verschlüsselte Programmpaket an einer Zwischenstation (z.B. einen Patientengerät, das zur Weiterleitung an ein Implantat ausgebildet ist) zwischengespeichert wird, bis sich eine Möglichkeit zur Übermittlung des Programmpakets an das persönliche Gerät ergibt (z.B. wenn das Implantat in den Empfangs- und Sendebereich des Patientengerätes gelangt). Das persönliche Gerät empfängt nun das verschlüsselte Programmpaket und kann es direkt einsetzen, ohne dass eine weitere Abstimmung mit dem Absender nötig wäre. Alternativ oder ergänzend kann allerdings auch eine Abfrage der Konfiguration der Datenübertragungsschnittstelle direkt an das persönliche Gerät selbst gerichtet sein, die das persönliche Gerät zu einer Rückmeldung veranlasst, der das Kennzeichen des jeweilig aktuellen Datenübertragungsprotokolls zu entnehmen ist. Insbesondere ist eine Datenbank vorteilhaft, deren Inhalt durch gelegentliche oder regelmäßige Abfragen an die persönlichen Geräte überprüft und verifiziert wird.

Gemäß einer vorteilhaften Ausführungsform der vorliegenden Erfindung ist die Speichereinheit zur Speicherung von allen Datenübertragungsprotokollen der Mehrzahl von Datenübertragungsprotokolle ausgebildet. Ist die Verwaltungseinheit über die Speichereinheit mit allen möglichen Datenübertragungsprotokollen ausgestattet, so ist damit gewährleistet, dass für jedes sich bei der Abfrage ergebende Datenübertragungsprotokoll ein kompatibles Datenübertragungsprotokoll zum Zurverfügungstellen findet.

Gemäß einer weiteren vorteilhaften Ausführungsform weist die Verwaltungseinheit ferner eine programmierbare Datenübertragungsschnittstelle zur Datenübertragung von und zu einem persönlichen Gerät auf, die mit einem mit dem Datenübertragungsprotokoll des persönlichen Gerätes kompatiblen Datenübertragungsprotokoll versehbar ist. Die Verwaltungseinheit ist mit einer eigenen Datenübertragungsschnittstelle ausgestattet, mit der eine Datenübertragung mit (d.h. zu und/oder von) dem persönlichen Gerät gemäß einen Datenübertragungsprotokoll ausgeführt werden kann. Damit die Verwaltungseinheit mit allen persönlichen Geräten kommunizieren kann, ist die Datenübertragungsschnittstelle der Verwaltungseinheit so ausgestaltet, das damit jeweils zumindest ein passend zu dem jeweiligen persönlichen Gerät ausgewähltes Datenübertragungsprotokoll ausgeführt werden kann.

Gemäß einer bevorzugten Ausführungsform sind die Datenübertragungsschnittstellen von Verwaltungseinheit und persönlichem Gerät ausgebildet, bei einer Übermittlung eines Datenübertragungsprotokolls an die Steuereinheit des persönlichen Gerätes das Datenübertragungsprotokoll des persönlichen Gerätes, ein damit kompatibles Datenübertragungsprotokoll und/oder ein anderes, vorbestimmtes Datenübertragungsprotokoll zu verwenden. Die Übertragung des Datenübertragungsprotokolls an das persönliche Gerät erfolgt entweder unter Einsatz bzw. gemäß dem derzeit in dem persönlichen Gerät zur allgemeinen Verwendung zur Verfügung stehenden Datenübertragungsprotokolls, das bei einer erfolgreichen Übertragung durch das neue Datenübertragungsprotokoll ersetzt oder ergänz wird, oder durch ein weiteres, bevorzugt speziell für dieses Zwecke bestimmtes Datenübertragungsprotokoll.

Gemäß einer Ausführungsform der Erfindung umfasst ein Zurverfügungstellen, für das die Verwaltungseinheit ausgebildet ist, ein Übermitteln des kompatiblen Datenübertragungsprotokolls an das externe Gerät und/oder ein Entgegennehmen und Weiterleiten wenigstens einer Datenübertragung von dem externen Gerät an das persönliche Gerät entsprechend dem kompatiblen Datenübertragungsprotokoll. Vorteilhafterweise umfasst das Zurverfügungstellen ein Entgegennehmen und Weiterleiten von Datenübertragungen von dem externen Gerät an das persönliche Gerät und von dem persönlichen Gerät an das externe Gerät. Eine erfindungsgemäße Möglichkeit besteht in der Übergabe des Datenübertragungsprotokolls an das externe Gerät. Das externe Gerät wird also selbst mit dem Datenübertragungsprotokoll ausgestattet. Eine andere, alternative oder ergänzende Möglichkeit besteht darin, dass die Verwaltungseinheit gewissermaßen als Relais-Station zwischen dem externen Gerät und dem persönlichen Gerät fungiert, wobei die Verwaltungseinheit sicherstellt, dass die Kommunikation mit dem persönlichen Gerät gemäß dem passenden Datenübertragungsprotokoll erfolgt.

In einer vorteilhaften Ausführungsform ist wenigstens ein Patientengerät zur Entgegennahme und Weiterleitung von Datenübertragungen zu und von wenigstens einem persönlichen Gerät vorgesehen. Das Patientengerät ist hierbei zum Empfang einer Datenübertragung von der Verwaltungseinheit und/oder einem externen Gerät ausgelegt, wobei die empfangenen Daten in verarbeiteter Form oder unverändert an das persönliche Gerät weitergeleitet werden. Entsprechendes ist auch für eine Übertragung in Gegenrichtung vorgesehen.

Gemäß einer weiteren Ausführungsform der Erfindung ist die Verwaltungseinheit ausgebildet, bei Bereitstellung eines neuen Datenübertragungsprotokolls in der Speichereinheit das neue Datenübertragungsprotokoll an alle oder an auf Basis eines oder mehrerer vorbestimmter Kriterien ausgewählte Steuereinheiten zur Änderung des Datenübertragungsprotokolls der Datenübertragungsschnittstellen der persönlichen Geräte zu übermitteln, wobei das oder die Kriterien insbesondere ausgewählt sind aus der Gruppe umfassend Identifikation des persönlichen Gerätes, Typ des persönlichen Gerätes, Kommunikationsweg zum persönlichen Gerät, Kennzeichen des Datenübertragungsprotokolls des persönlichen Gerätes, Standort des persönlichen Gerätes und Kombinationen davon. Beim Einspielen eines neuen Datenübertragungsprotokolls in die Speichereinheit der Verwaltungseinheit wird die Verwaltungseinheit veranlasst, dieses aktualisierte Datenübertragungsprotokoll - sofern dies vorgesehen ist - an persönliche Geräte zur Verwendung in deren Datenübertragungsschnittstelle zu übertragen. Somit werden bei einer Aktualisierung der Speichereinheit bevorzugt in automatisierter Weise auch die persönlichen Gerät mit aktualisierten Fassungen ausgestattet.

Kriterien, anhand derer von der Verwaltungseinheit entschieden wird, ob und wann eine Übertragung an etwa ein Implantat als persönlichem Gerät erfolgt, können beispielsweise sein:
- sobald sich ein Implantat zum Zwecke der Datenübertragung meldet, wird es über die Verfügbarkeit eines neuen Verschlüsselungs-/ Entschlüsselungs-Moduls informiert und mit diesem versorgt oder
- alle Implantate werden sofort mit dem neuen Verschlüsselungs- /Entschlüsselungs-Modul versorgt oder
- nach Seriennummern(kreisen) werden bestimmte Implantate mit dem neuen Verschlüsselungs-/Entschlüsselungs-Modul versorgt oder
- nach Implantatstyp werden die Implantate mit dem neuen Verschlüsselungs-/Entschlüsselungs-Modul versorgt,
- der Provider, über den sich das Implantat meldet,
- der Firmware Stand mit dem sich das Implantat meldet,
- die Region/Land/Zulassungsregion,
- nach einer Liste der Implantate, in denen diese nach Priorität für einen Update aufgeführt sind,
- der Typ des Patientengerätes, über den sich das Implantat meldet
- das Programm, in dem das Implantat läuft.

Gemäß einer weiteren Ausführungsform ist das persönliche Gerät zur Übermittlung einer Benachrichtigung an die Verwaltungseinheit bei vorgenommener und/oder nicht vorgenommener Änderung des Datenübertragungsprotokolls ausgebildet, wobei die Verwaltungseinheit zur Bewirkung eines entsprechenden Datenbankeintrags ausgebildet ist. Das persönliche Gerät sendet eine Bestätigung einer erfolgreich vorgenommenen Aktualisierung bzw. Änderung des Datenübertragungsprotokolls und/oder eine Nachricht hinsichtlich eines Fehlschlags bei einer Aktualisierung oder Änderung an die Verwaltungseinheit in Antwort auf die Übermittlung einen neuen Datenübertragungsprotokolls. Auf diese Weise wird ein Fehleintrag in der Datenbank vermieden, der sich ergeben könnte, wenn schon nach Übersendung des neuen Datenübertragungsprotokolls bereits ein entsprechender Eintrag in der Datenbank, in der die Zuordnung von persönlichen Geräten und Datenübertragungsprotokollen nachgehalten wird, vorgenommen würde, ohne dass eine Bestätigung der erfolgten Anpassung des persönlichen Gerätes abgewartet würde.

Gemäß einer Ausführungsform der Erfindung ist das persönliche Gerät mit einem weiteren Datenübertragungsprotokoll aus der Mehrzahl der Datenübertragungsprotokolle ausgestattet ist, das bei einem Ausfall des Datenübertragungsprotokolls des persönlichen Gerätes an dessen Stelle tritt. Die Datenübertragungsschnittstelle ist mit wenigstens zwei Datenübertragungsprotokollen ausgestattet oder haben Zugang zu diesen zwei Protokollen, wobei eines des Protokolle als ein weiteres, vorbestimmtes Protokoll dazu dient, beispielsweise im Fall eines Ausfalls des zunächst vorgesehenen Hauptprotokolls eingesetzt zu werden. Ein solcher Ausfall könnte sich beispielsweise durch einen Reset des persönlichen Gerätes ergeben, bei dem das eigentlich vorgesehene und bevorzugte Hauptprotokoll verloren geht.

Weitere vorteilhafte Ausgestaltungen der Erfindung ergeben sich insbesondere durch Kombination von Merkmalen der Ansprüche sowie aus der nachfolgenden Beschreibung bevorzugter Ausführungsbeispiele.

Die Erfindung wird im Folgenden anhand bevorzugter Ausführungsbeispiele mit Bezug auf die beiliegenden Figuren näher erläutert. Hierbei zeigt:
- Fig. 1: eine schematische Darstellung eines ersten Aspektes eines Ausführungsbeispiels der Erfindung,
- Fig. 2: eine schematische Ansicht der Verwaltungseinheit aus Fig. 1,
- Fig. 3: zeigt eine schematische Ansicht des Implantats aus Fig. 1,
- Fig. 4: eine schematische Darstellung eines zweiten Aspektes eines Ausführungsbeispiels der Erfindung, und
- Fig. 5: eine schematische Darstellung eine erfindungsgemäßen Verfahrensablaufs.

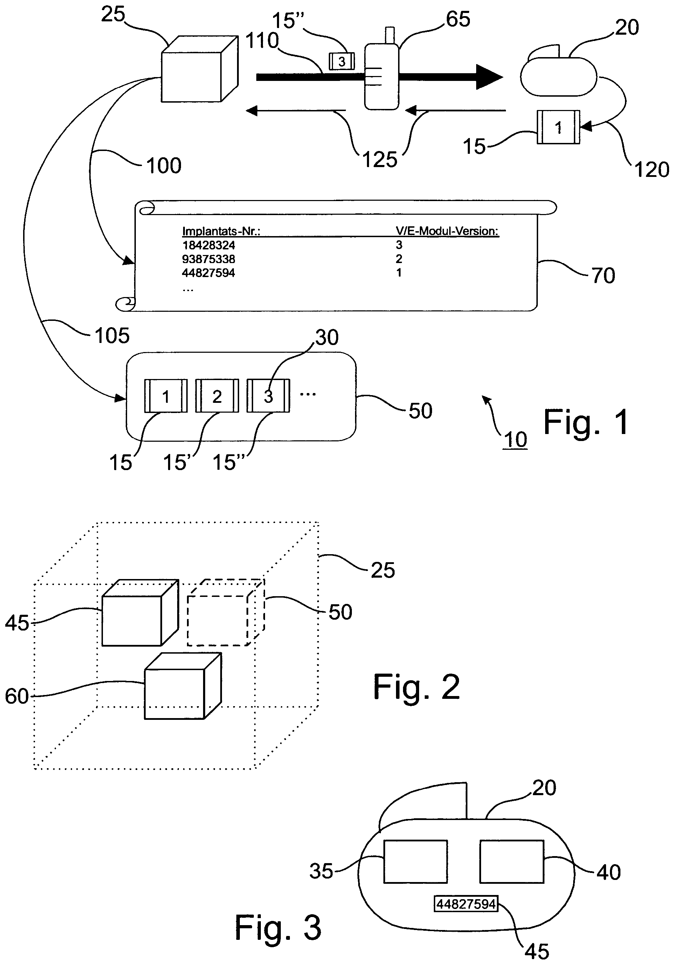

Fig. 1 zeigt eine schematische Darstellung eines ersten Aspektes eines Ausführungsbeispiels der Erfindung. Die Anordnung 10 umfasst eine Mehrzahl von Ver- und Entschlüsselungsmodulen 15, 15', 15" als Datenübertragungsprotokolle, eine Mehrzahl von Implantaten 20 als persönliche medizinische Gerät (wobei in Fig. 1 der Übersichtlichkeit halber nur ein Implantat 20 dargestellt ist), ein Patientengerät 65, eine Verwaltungseinheit 25 und eine Datenbank 70. Die Verwaltungseinheit 25 ist mit einer Speichereinheit 50 ausgestattet, die in der Darstellung von Fig. 1 außerhalb der Verwaltungseinheit 25 angeordnet ist. Die Speichereinheit 50 beinhaltet mehrere Ent- bzw. Verschlüsselungsmodule 15, 15', 15", wobei die Module 15, 15' und 15" jeweils mit einer Kennziffer 30 als Kennzeichen versehen sind. Die Datenbank 70 beinhaltet eine Zuordnung von Implantatsnummern zur Identifikation der Implantate zu den Kennziffern 30 oder Modulversionsnummern der Module 15, 15', 15". Die Verwaltungseinheit 25 hat jeweils Zugriff auf die Inhalte der Speichereinheit 50 und der Datenbank 70.

Fig. 2 zeigt eine schematische Ansicht der Verwaltungseinheit aus Fig. 1. Die Verwaltungseinheit 25 (hier punktiert dargestellt) umfasst eine Abfrageeinheit 45 und eine Datenübertragungsschnittstelle 60. In der Darstellung von Fig. 2 ist mit der gestrichelten Darstellung einer Speichereinheit 50 als Bestandteil der Verwaltungseinheit 25 eine alternative oder ergänzende Möglichkeit der Anordnung der Speichereinheit 50 im Vergleich zur Darstellung in Fig. 1 angedeutet.

Fig. 3 zeigt eine schematische Ansicht des Implantats aus Fig. 1. Das Implantat 20 umfasst eine Datenübertragungsschnittstelle 35, eine Steuereinheit 46 und eine Identifikation 45 in Form einer Implantatsnummer.

Unter Bezug auf die Fig. 1 bis 3 stellt die Verwaltungseinheit 25, die hier auch als Remote-Programming-Server bezeichnet werden kann, durch eine Abfrage der Datenbank 70 fest, dass das Implantat 20 ein Ver-/Entschlüsselungsmodul 15 mit der Kennziffer (1) verwendet. Diese Abfrage ist mit dem Pfeil 100 angedeutet und erfolgt unter Verwendung der Identifikation 45 des Implantats 20. Dieses Modul 15 stellt nicht den neuesten Stand dar, da bereits ein Modul 15" mit einer höheren Kennziffer (3) verfügbar ist. Aus der Speichereinheit 50, die hier auch als (V/E-Modul-)Repository bezeichnet werden kann, stellt der Remote-Programming-Server 25 das neuere Modul 15" bereit. Dies ist mit dem Pfeil 105 angedeutet. Wie mit dem Pfeil 110 angedeutet, sendet der Remote-Programming-Server 25 das Modul 15" an das Implantat 20, genauer an die Steuereinheit 40 des Implantats 20. Hierbei fungiert das auf das Implantat 20 abgestimmte Patientengerät 65 als Relais-Station und leitet die Übertragung vom Remote-Programming-Server 25 an das Implantat 20 weiter. Das Implantat 20, bzw. wiederum genauer dessen Steuereinheit 40, richtet die programmierbare Datenübertragungsschnittstelle 35 mit dem neuen Modul 15" an Stelle des alten Modul 15 ein, was durch den Pfeil 120 angedeutet ist. Je nach Ergebnis dieser Einrichtung berichtet das Implantat 20 - wiederum via Patientengerät 65 - über die erfolgreiche (oder auch erfolglose) Übernahme des neuen Moduls 15", was durch die Pfeile 125 angedeutet ist.

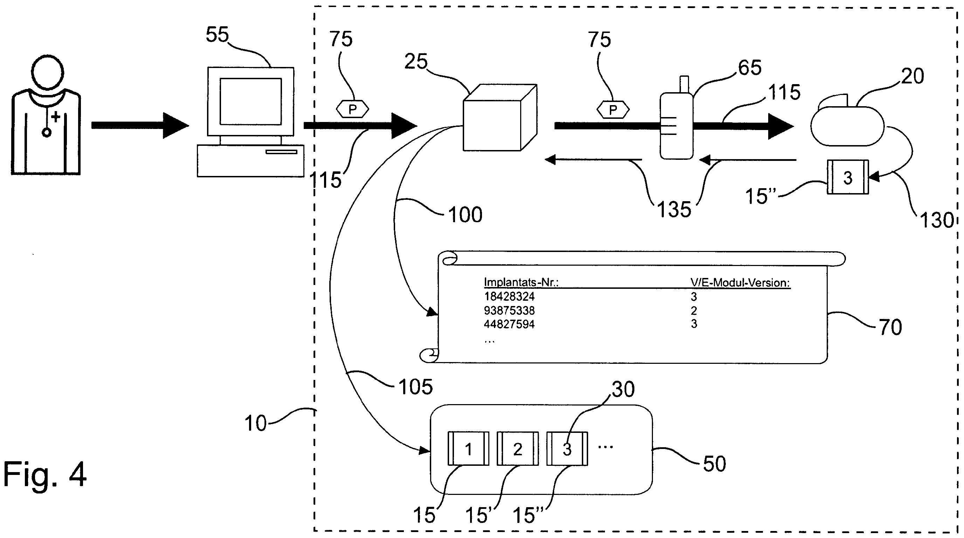

Fig. 4 zeigt eine schematische Darstellung eines zweiten Aspektes eines Ausführungsbeispiels der Erfindung. Die Anordnung 10 entspricht in der Darstellung von Fig. 4 im Wesentlichen der in Fig. 1 gezeigten Anordnung, wobei auch gilt, dass die Verwaltungseinheit 25 und das Implantat 20 in den Fig. 2 und 3 detaillierter dargestellt sind.

In Fig. 4 ist zusätzlich zu der Anordnung 10 ein Computer 55 als externes Gerät dargestellt, auf das ein Arzt Zugriff hat. Der Arzt erstellt in den Computer 55 ein Paket 75 zur Umprogrammierung des Implantats 20, beispielsweise mit einem geänderten Parametersatz zum Betrieb des Implantats 20. Das Paket 75 wird an den Remote-Programming-Server 25 gesendet, der über seine Abfrageeinheit 45 eine Abfrage 100 des von dem beabsichtigten Implantat 20 verwendeten Moduls an die Datenbank richtet. Diese Abfrage 100 ergibt, dass das Modul 15" mit der mit dem Kennzeichen (3) verwendet wird. Der Remote-Programming-Server 25 beschafft aus dem Speicher das Modul 15" und stattet seine Datenübertragungsschnittstelle 60 damit aus, das eine Verschlüsselung des Pakets 75 vornimmt. Das verschlüsselte Paket 75 wird vom Remote-Programming-Server 25 über das Patientengerät 65 an das Implantat 20 gesendet. Das Implantat 20 empfängt mit seiner Datenübertragungsschnittstelle 35 das Paket 75 und entschlüsselt es unter Verwendung seines Moduls 15" (angedeutet durch Pfeil 130). Ähnlich zu dem oben beschriebenen Ablauf bestätigt das Implantat 20 über das Patientengerät 65 die erfolgreiche (oder auch erfolglose) Übernahme des neuen Programms aus dem Paket 75 (angedeutet mit Pfeilen 135).

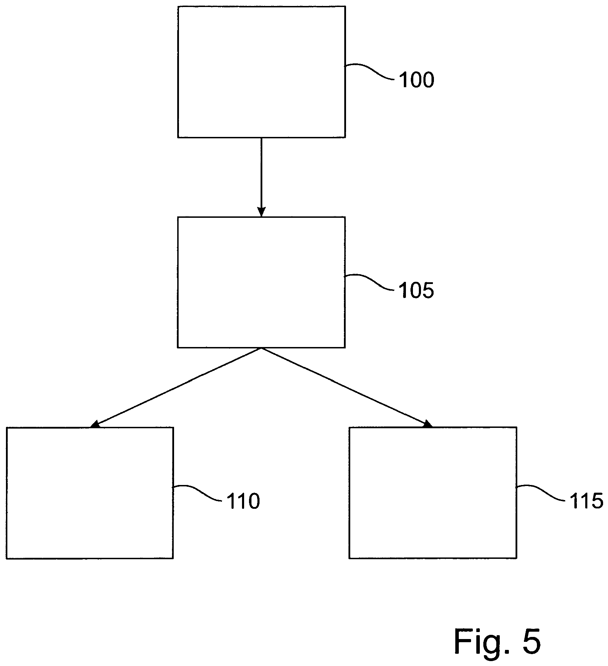

Fig. 5 zeigt eine schematische Darstellung eines erfindungsgemäßen Verfahrensablaufs. In Schritt 100 erfolgt ein Abfragen eines Kennzeichens eines Datenübertragungsprotokolls eines persönlichen Gerätes aus einer Mehrzahl von persönlichen Geräten auf Basis einer Identifikation des persönlichen Gerätes, wobei das Datenübertragungsprotokoll einer Mehrzahl von Datenübertragungsprotokollen angehört und wobei jedes Datenübertragungsprotokoll aus der Mehrzahl von Datenübertragungsprotokollen mit einem Kennzeichen versehen ist, das ausgebildet ist, das Datenübertragungsprotokoll von wenigstens einem anderen Datenübertragungsprotokoll aus der Mehrzahl von Datenübertragungsprotokollen zu unterscheiden. In Schritt 105 erfolgt auf Basis des Schritt 100 ein Bereitstellen von wenigstens einem Datenübertragungsprotokoll aus der Mehrzahl von Datenübertragungsprotokollen, das mit dem Datenübertragungsprotokoll des persönlichen Gerätes kompatibel ist oder das zum Ändern eines Datenübertragungsprotokoll eines persönliches Gerätes vorgesehen ist. In Schritt 110 erfolgt ein Übermitteln eines auf Basis der Kennzeichenabfrage bestimmten Datenübertragungsprotokolls aus der Speichereinheit an die Steuereinheit des persönlichen Gerätes zur Änderung des Datenübertragungsprotokolls der Datenübertragungsschnittstelle, während als Alternative dazu oder als Ergänzung in Schritt 115 ein Zurverfügungstellen des mit dem Datenübertragungsprotokoll des persönlichen Gerätes kompatibles Datenübertragungsprotokolls auf Basis der Kennzeichenabfrage für ein externes Gerät erfolgt.

Ein weiteres erfindungsgemäßes Ausführungsbeispiel umfasst eine Anwendung für einen Arzt, der ein Implantat umprogrammieren möchte, die dieser auf einem handelsüblichen Computer (externes Gerät) seiner Wahl ausführen kann. Mit Hilfe dieser Anwendung stellt der Arzt eine Programmierung für das Implantat (persönliches Gerät) zusammen und überträgt diese über eine geeignete Datenleitung (Internet, GPRS, GSM, UMTS o.ä.) an den Remote-Programming-Server (Verwaltungseinheit). Der Remote-Programming-Server umfasst ein Repository (Speichereinheit) für Verschlüsselungs-/Entschlüsselungs-Module (Datenübertragungsprotokolle), eine Liste (Datenbank), auf der vermerkt ist, welches Implantat derzeit über welches Verschlüsselungs-/Entschlüsselungs-Modul kommuniziert, und eine Vorrichtung oder Schnittstelle zum Kommunizieren mit der Arzt-Anwendung. Der Remote-Programming-Server hat die Fähigkeit, mit Hilfe eines der Verschlüsselungs-/Entschlüsselungs-Module ein Implantat-Programm verschlüsseln zu können, und die Fähigkeit, ein verschlüsseltes Implantat-Programm über eine geeignete Datenleitung (Internet, GPRS, GSM, UMTS o.ä.) weiterzusenden an ein Patientengerät in der Nähe des Implantats, das umprogrammiert werden soll. Dieses Patientengerät leitet das Umprogrammier-Datenpaket weiter in das Implantat. Das Implantat weist ein laufendes Implantat-Programm, nach welchem die durch das Implantat an den Patienten abgegebenen Therapien bestimmt werden, ein Verschlüsselungs-/Entschlüsselungs-Modul, über welches empfangene Daten entschlüsselt werden können (und zu versendende Daten verschlüsselt werden können), und eine Vorrichtung auf, die ausgebildet ist, ein entschlüsseltes Implantat-Programm auf Gültigkeit zu prüfen.

Bei diesem Ausführungsbeispiel arbeiten die Komponenten der Anordnung wie folgt zusammen:
Einerseits kann ein Update des Verschlüsselungs-/Entschlüsselungs-Moduls im Implantat vorgesehen sein. Der Remote-Programming-Server führt eine Liste darüber, welche Implantate derzeit welches Verschlüsselungs-/Entschlüsselungs-Modul enthalten. Sobald dem Remote-Programming-Server ein neues Paar Verschlüsselungs-/Entschlüsselungs-Module im Repository hinterlegt werden (der erste Teil des Paars zur Verwendung auf Seiten des Remote-Programming-Servers, der zweite Teil des Paars zur Verwendung auf Seiten des Implantats) beginnt der Remote-Programming-Server, diese nach bestimmten Kriterien an alle Implantate weiterzugeben, sodass deren Verschlüsselungs-/Entschlüsselungs-Module auf den neuesten Stand der Technik gebracht werden. Die Implantate prüfen jeweils bei Erhalt eines neuen Verschlüsselungs-/Entschlüsselungs-Moduls dessen Gültigkeit und setzen es dann in Betrieb. Darüber hinaus informieren sie den Remote-Programming-Server mittels einer geeigneten Quittung entweder bei ihrer nächsten regulären Datenübertragung oder sofort bei erfolgreicher Inbetriebnahme des erhaltenen Verschlüsselungs-/Entschlüsselungs-Moduls über die aktuell im Implantat verwendete Version des Verschlüsselungs-/Entschlüsselungs-Moduls. Der Remote-Programming-Server vermerkt dies auf seiner Liste (in der Datenbank). Falls das Implantat (beispielsweise bei einem Reset) auf ein Fallback-Programm zurückwechselt, welches auch ein Fallback-Verschlüsselungs-/Entschlüsselungs-Modul beinhaltet, so informiert es den Remote-Programming-Server auf geeignete Weise. Dies kann wiederum entweder bei seiner nächsten regulären Datenübertragung oder sofort erfolgen, sodass gegebenenfalls ein erneuter Versuch zum Update vorgenommen werden kann.

Andererseits kann eine Umprogrammierung des Implantats vorgesehen sein. Der Remote-Programming-Server erhält hierbei von der Arzt-Anwendung den Auftrag, die Programmierung eines bestimmten Implantats zu ändern. Der Remote-Programming-Server bestimmt mit Hilfe der hinterlegten Liste, welches Verschlüsselungs-/Entschlüsselungs-Modul auf demjenigen Implantat, welches umprogrammiert werden soll, derzeit eingesetzt wird. Der Remote-Programming-Server verschlüsselt das von der Arzt-Anwendung erhaltene Umprogrammier-Paket mit dem zuvor bestimmten Verschlüsselungs-/Entschlüsselungs-Modul und sendet es (beispielsweise via Patientengerät) in das Implantat. Das Implantat entschlüsselt das Umprogrammier-Paket und setzt es nach Prüfung auf Gültigkeit in Betrieb. Darüber hinaus informiert das Implantat den Remote-Programming-Server mittels einer geeigneten Quittung entweder bei seiner nächsten regulären Datenübertragung oder sofort bei erfolgreicher/erfolgloser Inbetriebnahme des neuen Programms über den Status der Umprogrammierung. Falls das Implantat (beispielsweise bei einem Reset) auf ein Fallback-Programm zurückwechselt, informiert es den Remote-Programming-Server darüber in geeigneter Weise. Dies kann wiederum entweder bei seiner nächsten regulären Datenübertragung oder sofort stattfinden.

dem Remote-Programming-Server ein neues Paar Verschlüsselungs-/Entschlüsselungs-Module im Repository hinterlegt werden (der erste Teil des Paars zur Verwendung auf Seiten des Remote-Programming-Servers, der zweite Teil des Paars zur Verwendung auf Seiten des Implantats) beginnt der Remote-Programming-Server, diese nach bestimmten Kriterien an alle Implantate weiterzugeben, sodass deren Verschlüsselungs-/Entschlüsselungs-Module auf den neuesten Stand der Technik gebracht werden. Die Implantate prüfen jeweils bei Erhalt eines neuen Verschlüsselungs-/Entschlüsselungs-Moduls dessen Gültigkeit und setzen es dann in Betrieb. Darüber hinaus informieren sie den Remote-Programming-Server mittels einer geeigneten Quittung entweder bei ihrer nächsten regulären Datenübertragung oder sofort bei erfolgreicher Inbetriebnahme des erhaltenen Verschlüsselungs-/Entschlüsselungs-Moduls über die aktuell im Implantat verwendete Version des Verschlüsselungs-/Entschlüsselungs-Moduls. Der Remote-Programming-Server vermerkt dies auf seiner Liste (in der Datenbank). Falls das Implantat (beispielsweise bei einem Reset) auf ein Fallback-Programm zurückwechselt, welches auch ein Fallback-Verschlüsselungs-/Entschlüsselungs-Modul beinhaltet, so informiert es den Remote-Programming-Server auf geeignete Weise. Dies kann wiederum entweder bei seiner nächsten regulären Datenübertragung oder sofort erfolgen, sodass gegebenenfalls ein erneuter Versuch zum Update vorgenommen werden kann.

Andererseits kann eine Umprogrammierung des Implantats vorgesehen sein. Der Remote-Programming-Server erhält hierbei von der Arzt-Anwendung den Auftrag, die Programmierung eines bestimmten Implantats zu ändern. Der Remote-Programming-Server bestimmt mit Hilfe der hinterlegten Liste, welches Verschlüsselungs-/Entschlüsselungs-Modul auf demjenigen Implantat, welches umprogrammiert werden soll, derzeit eingesetzt wird. Der Remote-Programming-Server verschlüsselt das von der Arzt-Anwendung erhaltene Umprogrammier-Paket mit dem zuvor bestimmten Verschlüsselungs-/Entschlüsselungs-Modul und sendet es (beispielsweise via Patientengerät) in das Implantat. Das Implantat entschlüsselt das Umprogrammier-Paket und setzt es nach Prüfung auf Gültigkeit in Betrieb. Darüber hinaus informiert das Implantat den Remote-Programming-Server mittels einer geeigneten Quittung entweder bei seiner nächsten regulären Datenübertragung oder sofort bei erfolgreicher/erfolgloser Inbetriebnahme des neuen Programms über den Status der Umprogrammierung. Falls das Implantat (beispielsweise bei einem Reset) auf ein Fallback-Programm zurückwechselt, informiert es den Remote-Programming-Server darüber in geeigneter Weise. Dies kann wiederum entweder bei seiner nächsten regulären Datenübertragung oder sofort stattfinden.

## Patentansprüche

1. Anordnung (10) zur Verwaltung einer Datenübertragungsschicht für ein implantierbares programmierbares persönliches medizinisches Gerät (20), mit den Komponenten:
- Mehrzahl von Ver- und Entschlüsselungsprotokollen sowie Authentifizierungsprotokollen als Datenübertragungsprotokolle (15, 15', 15"), die eine Mehrzahl von Datenübertragungsprotokollen (15, 15', 15") bilden,
- Mehrzahl von implantierbaren programmierbaren persönlichen medizinischen Geräten (20) und
- eine Verwaltungseinheit (25),
von denen jedes Datenübertragungsprotokoll (15, 15', 15") der Mehrzahl von Datenübertragungsprotokollen (15, 15', 15")
ein Kennzeichen (30) aufweist, das ausgebildet ist, das Datenübertragungsprotokoll (15, 15', 15") von wenigstens einem anderen Datenübertragungsprotokoll (15, 15', 15") aus der Mehrzahl von Datenübertragungsprotokollen (15, 15', 15") zu unterscheiden,
von denen jedes persönliches Gerät (20) der Mehrzahl von implantierbaren programmierbaren persönlichen medizinischen Geräten (20)
eine programmierbare Datenübertragungsschnittstelle (35) für eine Datenübertragung von und zum persönlichen Gerät (20) mit einem Datenübertragungsprotokoll (15, 15', 15") aus der Mehrzahl von Datenübertragungsprotokollen (15, 15', 15"),
eine Steuereinheit (40) zur Änderung des Datenübertragungsprotokolls (15, 15', 15") der programmierbaren Datenübertragungsschnittstelle (35), wobei die Steuereinheit (40) mindestens dazu ausgebildet ist, eine Datenübertragung über die Datenübertragungsschnittstelle (35) entgegenzunehmen, und
eine Identifikation (45), die das persönliche Gerät (20) kennzeichnet, aufweist,
von denen die Verwaltungseinheit (25)
eine Abfrageeinheit (45) für eine Abfrage des Kennzeichens (30) des Datenübertragungsprotokolls (15, 15', 15") eines persönlichen Gerätes (20) auf Basis der Identifikation (30) des persönlichen Gerätes (20) und
eine Speichereinheit (50) zur Speicherung von wenigstens einem Datenübertragungsprotokoll (15, 15', 15") aus der Mehrzahl von Datenübertragungsprotokollen (15, 15', 15") aufweist,
**dadurch gekennzeichnet, dass** die Verwaltungseinheit (25) ausgebildet ist,
- auf Basis der Kennzeichenabfrage einem externen Gerät (55) ein mit dem Datenübertragungsprotokoll (15, 15', 15") des persönlichen Gerätes (20) kompatibles Datenübertragungsprotokoll (15, 15', 15") aus der Speichereinheit (50) zur Verfügung zu stellen, und/oder
- ein auf Basis der Kennzeichenabfrage (100) bestimmtes Datenübertragungsprotokoll (15, 15', 15") aus der Speichereinheit (50) an die Steuereinheit (40) des persönlichen Gerätes (20) zur Änderung des Datenübertragungsprotokolls (15, 15', 15") der Datenübertragungsschnittstelle (35) zu übermitteln.

2. Anordnung (10) nach Anspruch 1, **dadurch gekennzeichnet, dass** wenigstens ein persönliches Gerät (20) der Mehrzahl von implantierbaren programmierbaren persönlichen medizinischen Geräten (20) ein aktives medizinisches Implantat (20) ist.

3. Anordnung (10) nach Anspruch 2, **dadurch gekennzeichnet, dass** das persönliche Gerät (20) ein implantierbarer Herzschrittmacher (20) oder Defibrillator-Kardioverter (20) ist.

4. Anordnung (10) nach Anspruch 1, **dadurch gekennzeichnet, dass** die Mehrzahl von Datenübertragungsprotokollen (15, 15', 15") erste Ver- und Entschlüsselungsprotokolle sowie Authentifizierungsprotokolle zur Verwendung in der Datenübertragungsschnittstelle (35) eines persönlichen Gerätes (20) und zweite Ver- und Entschlüsselungsprotokolle sowie Authentifizierungsprotokolle zur Verwendung beim Zurverfügungstellen durch die Verwaltungseinheit (25) aufweist.

5. Anordnung (10) nach einem der vorstehenden Ansprüche, **dadurch gekennzeichnet, dass** die Datenübertragungsschnittstelle (35) mit wenigstens zwei Datenübertragungsprotokollen versehen ist, insbesondere mit einem ersten Protokoll zur Verschlüsselung und einem zweiten Protokoll zur Entschlüsselung einer Datenübertragung.

6. Anordnung (10) nach einem der vorstehenden Ansprüche, **dadurch gekennzeichnet, dass** das Kennzeichen (30) eine Versionsnummer, ein Erstellungsdatum, ein Identifikationszeichen und/oder eine Klassifikation des Datenübertragungsprotokolls umfasst.

7. Anordnung (10) nach einem der vorstehenden Ansprüche, **dadurch gekennzeichnet, dass** die Abfrageeinheit (45)
- zur Abfrage des Kennzeichens (30) aus einer Datenbank (70) der Anordnung (10) auf Basis der Identifikation (45) des persönlichen Gerätes (20) und/oder
- zur Abfrage des Kennzeichens (30) durch Abfragen des persönlichen Gerätes (20) ausgebildet ist, wobei das persönliche Gerät (20) zur Beantwortung einer Abfrage der Abfrageeinheit (45) durch Übermittlung des Kennzeichens (30) ausgebildet ist.

8. Anordnung (10) nach einem der vorstehenden Ansprüche, **dadurch gekennzeichnet, dass** die Speichereinheit (50) zur Speicherung von allen Datenübertragungsprotokollen (15, 15', 15") der Mehrzahl von Datenübertragungsprotokolle (15, 15', 15") ausgebildet ist.

9. Anordnung (10) nach einem der vorstehenden Ansprüche, **dadurch gekennzeichnet, dass** die Verwaltungseinheit (25) ferner eine programmierbare Datenübertragungsschnittstelle (60) zur Datenübertragung von und zu einem persönlichen Gerät (20) aufweist, die mit einem mit dem Datenübertragungsprotokoll (15, 15', 15") des persönlichen Gerätes (20) kompatiblen Datenübertragungsprotokoll (15, 15', 15") versehbar ist.

10. Anordnung (10) nach einem der vorstehenden Ansprüche, **dadurch gekennzeichnet, dass** die Datenübertragungsschnittstellen (35, 60) von Verwaltungseinheit (25) und persönlichem Gerät (20) ausgebildet sind, bei einer Übermittlung eines Datenübertragungsprotokolls an die Steuereinheit (40) des persönlichen Gerätes das Datenübertragungsprotokoll (15, 15', 15") des persönlichen Gerätes, ein damit kompatibles Datenübertragungsprotokoll (15, 15', 15") und/oder ein anderes, vorbestimmtes Datenübertragungsprotokoll (15, 15', 15") zu verwenden.

11. Anordnung (10) nach einem der vorstehenden Ansprüche, **dadurch gekennzeichnet, dass** die Verwaltungseinheit für ein Zurverfügungstellen ausgebildet ist, das
- ein Übermitteln des kompatiblen Datenübertragungsprotokolls (15, 15', 15") an das externe Gerät (55)
und/oder
- ein Entgegennehmen und Weiterleiten wenigstens einer Datenübertragung von dem externen Gerät (55) an das persönliche Gerät (20) entsprechend dem kompatiblen Datenübertragungsprotokoll (15, 15', 15") umfasst, insbesondere ein Entgegennehmen und Weiterleiten von Datenübertragungen von dem externen Gerät (55) an das persönliche Gerät (20 und von dem persönlichen Gerät (20) an das externe Gerät (55).

12. Anordnung (10) nach einem der vorstehenden Ansprüche, **gekennzeichnet durch** wenigstens ein Patientengerät (65) zur Entgegennahme und Weiterleitung von Datenübertragungen zu und von wenigstens einem persönlichen Gerät (20).

13. Anordnung (10) nach einem der vorstehenden Ansprüche, **dadurch gekennzeichnet, dass** die Verwaltungseinheit (25) ausgebildet ist, bei Bereitstellung eines neuen Datenübertragungsprotokolls (15, 15', 15") in der Speichereinheit (50) das neue Datenübertragungsprotokoll (15, 15', 15") an alle oder an auf Basis eines oder mehrerer vorbestimmter Kriterien ausgewählte Steuereinheiten (40) zur Änderung des Datenübertragungsprotokolls (15, 15', 15") der Datenübertragungsschnittstellen (35) der persönlichen Geräte (20) zu übermitteln, wobei das oder die Kriterien insbesondere ausgewählt sind aus der Gruppe umfassend Identifikation (45) des persönlichen Gerätes (20), Typ des persönlichen Gerätes (20), Kommunikationsweg zum persönlichen Gerät (20), Kennzeichen (30) des Datenübertragungsprotokolls (15, 15', 15") des persönlichen Gerätes (20), Standort des persönlichen Gerätes (20) und Kombinationen davon.

14. Anordnung (10) nach einem der vorstehenden Ansprüche, **dadurch gekennzeichnet, dass** das persönliche Gerät (20) zur Übermittlung einer Benachrichtigung an die Verwaltungseinheit (25) bei vorgenommener und/oder nicht vorgenommener Änderung des Datenübertragungsprotokolls (15, 15', 15") ausgebildet ist, wobei die Verwaltungseinheit (25) zur Bewirkung eines entsprechenden Datenbankeintrags ausgebildet ist.

15. Anordnung (10) nach einem der vorstehenden Ansprüche, **dadurch gekennzeichnet, dass** das persönliche Gerät (20) mit einem weiteren Datenübertragungsprotokoll (15, 15', 15") aus der Mehrzahl der Datenübertragungsprotokolle (15, 15', 15") ausgestattet ist, das bei einem Ausfall des Datenübertragungsprotokolls (15, 15', 15") des persönlichen Gerätes (20) an dessen Stelle tritt.

16. Verwaltungseinheit (25) für eine Anordnung zur Verwaltung einer Datenübertragungsschicht für ein implantierbares programmierbares persönliches medizinisches Gerät (20), nach einem der Ansprüche 1 bis 15, mit den Komponenten:
- eine Abfrageeinheit (45)
und
- eine Speichereinheit (50),
von denen die Abfrageeinheit (45)
für eine Abfrage eines Kennzeichens (30) eines Datenübertragungsprotokolls (15, 15', 15") das ein Ver- oder Entschlüsselungsprotokoll oder ein Authentifizierungsprotokoll ist, einer programmierbaren Datenübertragungsschnittstelle (35) eines persönlichen Gerätes (20) aus einer Mehrzahl von implantierbaren programmierbaren persönlichen medizinischen Geräten (20) auf Basis einer Identifikation (45) des persönlichen Gerätes (20) ausgebildet ist, wobei Ver- und Entschlüsselungsprotokolle sowie Authentifizierungsprotokolle eine Mehrzahl von Datenübertragungsprotokollen (15, 15', 15") bilden, wobei das Kennzeichen (30) ausgebildet ist, das Datenübertragungsprotokoll (15, 15', 15") von wenigstens einem anderen Datenübertragungsprotokoll (15, 15', 15") aus einer Mehrzahl von Datenübertragungsprotokollen (15, 15', 15") zu unterscheiden, und
von denen die Speichereinheit (50)
zur Speicherung von wenigstens einem Datenübertragungsprotokoll (15, 15', 15") aus der Mehrzahl von Datenübertragungsprotokollen (15, 15', 15") ausgebildet ist,
**dadurch gekennzeichnet, dass** die Verwaltungseinheit (25) ausgebildet ist,
- auf Basis der Kennzeichenabfrage einem externen Gerät (55) ein mit dem Datenübertragungsprotokoll (15, 15', 15") des persönlichen Gerätes (20) kompatibles Datenübertragungsprotokoll (15, 15', 15") aus der Speichereinheit (50) zur Verfügung zu stellen, und/oder
- ein auf Basis der Kennzeichenabfrage bestimmtes Datenübertragungsprotokoll (15, 15', 15") aus der Speichereinheit (50) an eine Steuereinheit (40) des persönlichen Gerätes (20) zur Änderung des Datenübertragungsprotokolls (15, 15', 15") der Datenübertragungsschnittstelle (35) zu übermitteln.

17. Verfahren zur Verwaltung einer Datenübertragungsschicht für ein implantierbares programmierbares persönliches medizinisches Gerät (20), insbesondere mittels einer Anordnung (10) nach einem der Ansprüche 1 bis 15, mit den Verfahrensschritten:
- Abfragen (100) eines Kennzeichens (30) eines Datenübertragungsprotokolls (15, 15', 15") eines persönlichen Gerätes (20) aus einer Mehrzahl von implantierbaren programmierbaren persönlichen medizinischen Geräten (20) auf Basis einer Identifikation (45) des persönlichen Gerätes (20), wobei das Datenübertragungsprotokoll (15, 15', 15") ein Ver- oder Entschlüsselungsprotokoll oder ein Authentifizierungsprotokoll ist und einer Mehrzahl von Datenübertragungsprotokollen (15, 15', 15"), die Ver- und Entschlüsselungsprotokolle sowie Authentifizierungsprotokolle sind, angehört und wobei jedes Datenübertragungsprotokoll (15, 15', 15") aus der Mehrzahl von Datenübertragungsprotokollen (15, 15', 15") mit einem Kennzeichen (30) versehen ist, das ausgebildet ist, das Datenübertragungsprotokoll (15, 15', 15") von wenigstens einem anderen Datenübertragungsprotokoll (15, 15', 15") aus der Mehrzahl von Datenübertragungsprotokollen (15, 15', 15") zu unterscheiden,
- Bereitstellen (105) von wenigstens einem Datenübertragungsprotokoll (15, 15', 15") aus der Mehrzahl von Datenübertragungsprotokollen (15, 15', 15"), das mit dem Datenübertragungsprotokoll (15, 15', 15") des persönlichen Gerätes (20) kompatibel ist oder das zum Ändern eines Datenübertragungsprotokoll (15, 15', 15") eines persönliches Gerätes (20) vorgesehen ist,
**dadurch gekennzeichnet, dass** das Verfahren ferner als Verfahrensschritte aufweist:
- Zurverfügungstellen (115) des mit dem Datenübertragungsprotokoll (15, 15', 15") des persönlichen Gerätes (15, 15', 15") kompatiblen Datenübertragungsprotokolls (15, 15', 15") auf Basis der Kennzeichenabfrage (100) für ein externes Gerät (55)
und/oder
- Übermitteln (110) eines auf Basis der Kennzeichenabfrage (100) bestimmten Datenübertragungsprotokolls (15, 15', 15") an eine Steuereinheit (40) des persönlichen Gerätes (20) zur Änderung des Datenübertragungsprotokolls (15, 15', 15") der Datenübertragungsschnittstelle (35).

## Claims

1. An arrangement (10) for managing a data transmission layer for an implantable, programmable personal medical device (20), said arrangement comprising the following components:
- a plurality of encryption and decryption protocols and authentication protocols as data transmission protocols (15, 15', 15"), which form a plurality of data transmission protocols (15, 15', 15"),
- a plurality of implantable programmable personal medical devices (20), and
- a management unit (25),
each data transmission protocol (15, 15', 15") of the plurality of data transmission protocols (15, 15', 15")
having a designation (30) which is configured to distinguish the data transmission protocol (15, 15', 15") from at least one other data transmission protocol (15, 15', 15") from the plurality of data transmission protocols (15, 15', 15"),
each personal device (20) of the plurality of implantable programmable personal medical devices (20)
having a programmable data transmission interface (35) for data transmission from and to the personal device (20) with a data transmission protocol (15, 15', 15") from the plurality of data transmission protocols (15, 15', 15"),
a control unit (40) for changing the data transmission protocol (15, 15', 15") of the programmable data transmission interface (35), wherein the control unit (40) is configured at least to accept a data transmission via the data transmission interface (35), and
an identification (45), which identifies the personal device (20),
the management unit (25)
having a query unit (45) for querying the designation (30) of the data transmission protocol (15, 15', 15") of a personal device (20) on the basis of the identification (30) of the personal device (20) and
a memory unit (50) for storing at least one data transmission protocol (15, 15', 15") from the plurality of data transmission protocols (15, 15', 15"),
**characterised in that** the management unit (25) is configured
- to make available to an external device (55) from the memory unit (50) a data transmission protocol (15, 15', 15") compatible with the data transmission protocol (15, 15', 15") of the personal device (20) on the basis of the designation query, and/or
- to transmit a specific data transmission protocol (15, 15', 15") from the memory unit (50) to the control unit (40) of the personal device (20) on the basis of the designation query (100) in order to change the data transmission protocol (15, 15', 15") of the data transmission interface (35).

2. The arrangement (10) according to claim 1, **characterised in that** at least one personal device (20) of the plurality of implantable programmable personal medical devices (20) is an active medical implant (20).

3. The arrangement (10) according to claim 2, **characterised in that** the personal device (20) is an implantable cardiac pacemaker (20) or an implantable defibrillator-cardioverter (20).

4. The arrangement (10) according to claim 1, **characterised in that** the plurality of data transmission protocols (15, 15', 15") comprises first encryption and decryption protocols and authentication protocols for use in the data transmission interface (35) of a personal device (20) and second encryption and decryption protocols and authentication protocols for use when the management unit (25) makes available the compatible data transmission protocol.

5. The arrangement (10) according to any one of the preceding claims, **characterised in that** the data transmission interface (35) is provided with at least two data transmission protocols, in particular with a first protocol for encrypting a data transmission and a second protocol for decrypting a data transmission.

6. The arrangement (10) according to any one of the preceding claims, **characterised in that** the designation (30) comprises a version number, a creation date, an identification sign and/or a classification of the data transmission protocol.

7. The arrangement (10) according to any one of the preceding claims, **characterised in that** the query unit (45)
- is configured to query the designation (30) from a database (70) of the arrangement (10) on the basis of the identification (45) of the personal device (20) and/or
- to query the designation (30) by querying the personal device (20), wherein the personal device (20) is configured to respond to a query of the query unit (45) by transmission of the designation (30).

8. The arrangement (10) according to any one of the preceding claims, **characterised in that** the memory unit (50) is configured to store all data transmission protocols (15, 15', 15") of the plurality of data transmission protocols (15, 15', 15").

9. The arrangement (10) according to any one of the preceding claims, **characterised in that** the management unit (25) also has a programmable data transmission interface (60) for data transmission from and to a personal device (20), which interface can be provided with a data transmission protocol (15, 15', 15") compatible with the data transmission protocol (15, 15', 15") of the personal device (20).

10. The arrangement (10) according to any one of the preceding claims, **characterised in that** the data transmission interfaces (35, 60) of the management unit (25) and of the personal device (20) are configured, when transmitting a data transmission protocol to the control unit (40) of the personal device, to use the data transmission protocol (15, 15', 15") of the personal device, a data transmission protocol (15, 15', 15") compatible therewith, and/or another predetermined data transmission protocol (15, 15', 15").

11. The arrangement (10) according to any one of the preceding claims, **characterised in that** the management unit is configured to make available a compatible data transmission protocol, said process comprising
- transmitting the compatible data transmission protocol (15, 15', 15") to the external device (55) and/or
- accepting and forwarding at least one data transmission from the external device (55) to the personal device (20) in accordance with the compatible data transmission protocol (15, 15', 15"), in particular accepting and forwarding data transmissions from the external device (55) to the personal device (20) and from the personal device (20) to the external device (55).

12. The arrangement (10) according to any one of the preceding claims, **characterised by** at least one patient device (65) for accepting and forwarding data transmissions to and from at least one personal device (20).

13. The arrangement (10) according to any one of the preceding claims, **characterised in that** the management unit (25) is configured, when providing a new data transmission protocol (15, 15', 15") in the memory unit (50), to transmit the new data transmission protocol (15, 15', 15") to all or selected control units (40) on the basis of one or more predetermined criteria in order to change the data transmission protocol (15, 15', 15") of the data transmission interfaces (35) of the personal devices (20), wherein the one or more criteria is/are selected in particular from the group comprising identification (45) of the personal device (20), type of personal device (20), communication path to the personal device (20), designation (30) of the data transmission protocol (15, 15', 15") of the personal device (20), location of the personal device (20), and combinations thereof.

14. The arrangement (10) according to any one of the preceding claims, **characterised in that** the personal device (20) is configured to transmit a message to the management unit (25) when a change to the data transmission protocol (15, 15', 15") is implemented and/or not implemented, wherein the management unit (25) is configured to cause a corresponding database entry.

15. The arrangement (10) according to any one of the preceding claims, **characterised in that** the personal device (20) is equipped with a further data transmission protocol (15, 15', 15") from the plurality of data transmission protocols (15, 15', 15") which replaces the data transmission protocol (15, 15', 15") of the personal device (20) in the event of its failure.

16. A management unit (25) for an arrangement for managing a data transmission layer for an implantable programmable personal medical device (20) according to any one of claims 1 to 15, said management unit comprising the following components:
- a query unit (45) and
- a memory unit (50),
the query unit (45)
being configured to query a designation (30) of a data transmission protocol (15, 15', 15"), which is an encryption or decryption protocol or an authentication protocol, of a programmable data transmission interface (35) of a personal device (20) from a plurality of implantable programmable personal medical devices (20) on the basis of an identification (45) of the personal device (20), wherein encryption and decryption protocols and authentication protocols form a plurality of data transmission protocols (15, 15', 15"), wherein the designation (30) is configured to distinguish the data transmission protocol (15, 15', 15") from at least one other data transmission protocol (15, 15', 15") from a plurality of data transmission protocols (15, 15', 15"), and
the memory unit (50)
being configured to store at least one data transmission protocol (15, 15', 15") from the plurality of data transmission protocols (15, 15', 15"),
**characterised in that** the management unit (25) is configured
- to make available to an external device (55) from the memory unit (50) a data transmission protocol (15, 15', 15") compatible with the data transmission protocol (15, 15', 15") of the personal device (20) on the basis of the designation query, and/or
- to transmit a specific data transmission protocol (15, 15', 15") from the memory unit (50) to the control unit (40) of the personal device (20) on the basis of the designation query (100) in order to change the data transmission protocol (15, 15', 15") of the data transmission interface (35).

17. A method for managing a data transmission layer for an implantable programmable personal medical device (20), in particular by means of an arrangement (10) according to any one of claims 1 to 15, said method comprising the following method steps:
- querying (100) a designation (30) of a data transmission protocol (15, 15', 15") of a personal device (20) from a plurality of implantable programmable personal medical devices (20) on the basis of an identification (45) of the personal device (20), wherein the data transmission protocol (15, 15', 15") is an encryption or decryption protocol or an authentication protocol and belongs to a plurality of data transmission protocols (15, 15', 15") which are encryption and decryption protocols and authentication protocols, and wherein each data transmission protocol (15, 15', 15") from the plurality of data transmission protocols (15, 15', 15") is provided with a designation (30) which is configured to distinguish the data transmission protocol (15, 15', 15") from at least one other data transmission protocol (15, 15', 15") from the plurality of data transmission protocols (15, 15', 15"),
- providing (105) at least one data transmission protocol (15, 15', 15") from the plurality of data transmission protocols (15, 15', 15") which is compatible with the data transmission protocol (15, 15', 15") of the personal device (20) or which is intended to change a data transmission protocol (15, 15', 15") of a personal device (20),
**characterised in that** the method also comprises the following as method steps:
- making available (115) for an external device (55) the data transmission protocol (15, 15', 15") compatible with the data transmission protocol (15, 15', 15") of the personal device (20) on the basis of the designation query (100) and/or
- transmitting (110) a data transmission protocol (15, 15', 15") on the basis of the designation query (100) to a control unit (40) of the personal device (20) in order to change the data transmission protocol (15, 15', 15") of the data transmission interface (35).

## Revendications

1. Agencement (10) de gestion d'une couche de transmission de données pour un appareil médical personnel (20), implantable, programmable doté des composantes :
- une multiplicité de protocoles d'encryptage et de décryptage, ainsi que de protocoles d'authentification servant de protocoles de transmission de données (15, 15', 15") qui forment une multiplicité de protocoles de transmission de données (15, 15', 15"),
- une multiplicité d'appareils médicaux personnels (20), implantables, programmables, et
- une unité d'exploitation (25),
parmi lesquels, chaque protocole de transmission de données (15, 15', 15") de la multiplicité des protocoles de transmission de données (15, 15', 15")
présente un signe distinctif (30), qui est conçu pour distinguer le protocole de transmission de données (15, 15', 15") d'au moins un autre protocole de transmission de données (15, 15', 15") parmi la multiplicité de protocoles de transmission de données (15, 15', 15"),
parmi lesquels, chaque appareil personnel (20) de la multiplicité des appareils médicaux personnels (20), implantables, programmables, présente
une interface de transmission de données (35) programmable pour une transmission de données au départ et en direction de l'appareil personnel (20) avec un protocole de transmission de données (15, 15', 15") parmi la multiplicité de protocoles d transmission de données (15, 15', 15"),
une unité de commande (40) pour la modification du protocole de transmission de données (15, 15', 15") de l'interface de transmission de données (35) programmable, où l'unité de commande (40) est conçue au moins pour accepter une transmission de données par le biais de l'interface de transmission de données (35), et
une identification (45), qui caractérise l'appareil personnel (20),
parmi lesquels, l'unité d'exploitation (25) présente
une unité de questionnement (45) pour un questionnement concernant le signe distinctif (30) du protocole de transmission de données (15, 15', 15") d'un appareil personnel (20) sur la base de l'identification (30) de l'appareil personnel (20), et une unité de mémoire (50) pour l'enregistrement d'au moins un protocole de transmission de données (15, 15', 15") parmi la multiplicité de protocoles de transmission de données (15, 15', 15"),
**caractérisé en ce que** l'unité d'exploitation (25) est conçue pour
- sur la base du questionnement concernant le signe distinctif d'un appareil externe (55), mettre à disposition un protocole de transmission de données (15, 15', 15") à partir de l'unité de mémoire (50) compatible avec le protocole de transmission de données (15, 15', 15") de l'appareil personnel (20), et/ou
- transmettre un protocole de transmission de données (15, 15', 15") déterminé sur la base du questionnement (100) concernant le signe distinctif à partir de l'unité de mémoire (50) vers l'unité de commande (40) de l'appareil personnel (20) pour la modification du protocole de transmission de données (15, 15', 15") de l'interface de transmission de données (35).

2. Agencement (10) selon la revendication 1, **caractérisé en ce qu'**au moins un appareil personnel (20) de la multiplicité des appareils médicaux personnels (20), implantables, programmables, est un implant (20) médical actif.

3. Agencement (10) selon la revendication 2, **caractérisé en ce que** l'appareil personnel (20) est un stimulateur cardiaque (20) ou un défibrillateur cardioverteur (20) implantable.

4. Agencement (10) selon la revendication 1, **caractérisé en ce que** la multiplicité des protocoles de transmission de données (15, 15', 15") présente des premiers protocoles d'encryptage et de décryptage, ainsi que des protocoles d'authentification pour l'emploi dans l'interface de transmission de données (35) d'un appareil personnel (20) et des deuxièmes protocoles d'encryptage et de décryptage, ainsi que des protocoles d'authentification pour l'emploi lors de la mise à disposition par l'unité d'exploitation (25).

5. Agencement (10) selon l'une des revendications précédentes, **caractérisé en ce que** l'interface de transmission de données (35) est muni d'au moins deux protocoles de transmission de données, notamment d'un premier protocole pour l'encryptage et un deuxième protocole pour le décryptage d'une transmission de données.

6. Agencement (10) selon l'une des revendications précédentes, **caractérisé en ce que** le signe distinctif (30) comprend un numéro de version, une date d'édition, un caractère d'identification et/ou une classification du protocole de transmission de données.

7. Agencement (10) selon l'une des revendications précédentes, **caractérisé en ce que** l'unité de questionnement (45) est conçue
- pour le questionnement concernant le signe distinctif (30) à partir d'une banque de données (70) de l'agencement (10) sur la base de l'identification (45) de l'appareil personnel (20), et/ou
- pour le questionnement concernant le signe distinctif (30) par le questionnement de l'appareil personnel (20), où l'appareil personnel (20) est conçu pour répondre à une question de l'unité de questionnement (45) par la transmission du signe distinctif (30).

8. Agencement (10) selon l'une des revendications précédentes, **caractérisé en ce que** l'unité de mémoire (50) est conçue pour l'enregistrement de tous les protocoles de transmission de données (15, 15', 15") de la multiplicité des protocoles de transmission de données (15, 15', 15").

9. Agencement (10) selon l'une des revendications précédentes, **caractérisé en ce que** l'unité d'exploitation (25) présente en outre une interface de transmission de données programmable (60) pour la transmission de données au départ et en direction d'un appareil personnel (20), qui peut être munie d'un protocole de transmission de données (15, 15', 15") compatible avec le protocole de transmission de données (15, 15', 15") de l'appareil personnel (20).

10. Agencement (10) selon l'une des revendications précédentes, **caractérisé en ce que** les interfaces de transmission de données (35, 60) de l'unité d'exploitation (25) et de l'appareil personnel (20) sont conçues, lors d'une transmission d'un protocole de transmission de données à l'unité de commande (40) de l'appareil personnel, pour employer le protocole de transmission de données (15, 15', 15") de l'appareil personnel, un protocole de transmission de données (15, 15', 15") compatible avec celui-ci et/ou un autre protocole de transmission de données (15, 15', 15") prédéterminé.

11. Agencement (10) selon l'une des revendications précédentes, **caractérisé en ce que** l'unité d'exploitation est conçue pour une mise à disposition qui comprend
- une transmission du protocole de transmission de données (15, 15', 15") compatible vers l'appareil externe (55)
et/ou
- une acceptation et un transfert d'au moins une transmission de données de l'appareil externe (55) vers l'appareil personnel (20) selon le protocole de transmission de données (15, 15', 15") compatible, notamment une acceptation et un transfert de transmissions de données de l'appareil externe (55) vers l'appareil personnel (20), et de l'appareil personnel (20) vers l'appareil externe (55).

12. Agencement (10) selon l'une des revendications précédentes, **caractérisé par** au moins un appareil de patient (65) destiné à la réception et au transfert de transmissions de données en direction et au départ d'au moins d'un appareil personnel (20).

13. Agencement (10) selon l'une des revendications précédentes, **caractérisé en ce que** l'unité d'exploitation (25) est conçue pour, lors de la mise à disposition d'un nouveau protocole de transmission de données (15, 15', 15"), dans l'unité de mémoire (50), transmettre le nouveau protocole de transmission de données (15, 15', 15") à toutes ou à des unités de commande (40) choisies sur la base d'un ou de plusieurs critères prédéterminés pour la modification du protocole de transmission de données (15, 15', 15") des interfaces de transmission de données (35) des appareils personnels (20), où le ou les critères sont en particulier choisis dans le groupe comprenant une identification (45) de l'appareil personnel (20), un type de l'appareil personnel (20), une voie de communication vers l'appareil personnel (20), un signe distinctif (30) du protocole de transmission de données (15, 15', 15") de l'appareil personnel (20), un emplacement de l'appareil personnel (20), et de combinaisons de ceux-ci.

14. Agencement (10) selon l'une des revendications précédentes, **caractérisé en ce que** l'appareil personnel (20) est conçu pour la transmission d'une notification à l'unité d'exploitation (25) lors d'une modification du protocole de transmission de données (15, 15', 15") exécutée et/ou non exécutée, où l'unité d'exploitation (25) est conçue pour l'activation d'une entrée de banque de données correspondante.

15. Agencement (10) selon l'une des revendications précédentes, **caractérisé en ce que** l'appareil personnel (20) est équipé d'un autre protocole de transmission de données (15, 15', 15") parmi la multiplicité de protocoles de transmission de données (15, 15', 15") qui, lors d'une défaillance du protocole de transmission de données (15, 15', 15") de l'appareil personnel (20), prend la place de celui-ci.

16. Unité d'exploitation (25) destinée à un agencement pour l'exploitation d'une couche de transmission de données destinée à un appareil médical personnel (20), implantable, programmable, selon l'une des revendications 1 à 15, avec les composantes :
- une unité de questionnement (45)
et
- une unité de mémoire (50),
parmi lesquelles, l'unité de questionnement (45) est conçue
pour un questionnement concernant un signe distinctif (30) d'un protocole de transmission de données (15, 15' 15") qui est un protocole d'encryptage ou de décryptage, ou un protocole d'authentification, d'une interface de transmission de données (35) programmable d'un appareil personnel (20) parmi une multiplicité d'appareils médicaux personnels (20), implantables, programmables sur la base d'une identification (45) de l'appareil personnel (20), où les protocoles d'encryptage et de décryptage, ainsi que les protocoles d'authentification, forment une multiplicité de protocoles de transmission de données (15, 15', 15"), où le signe distinctif (30) est conçu pour différencier le protocole de transmission de données (15, 15', 15") d'au moins un autre protocole de transmission de données (15, 15', 15") parmi une multiplicité de protocoles de transmission de données (15, 15', 15"), et
parmi lesquelles, l'unité de mémoire (50) est conçue
pour l'enregistrement d'au moins un protocole de transmission de données (15, 15', 15") parmi une multiplicité de protocoles de transmission de données (15, 15', 15"),
**caractérisée en ce que** l'unité d'exploitation (25) est conçue pour,
- sur la base du questionnement concernant le signe distinctif d'un appareil externe (55), mettre à disposition un protocole de transmission de données (15, 15', 15") compatible avec le protocole de transmission de données (15, 15', 15") de l'appareil personnel (20), à partir de l'unité de mémoire (50), et/ou pour
- transmettre, à partir de l'unité de mémoire (50) à une unité de commande (40) de l'appareil personnel (20), un protocole de transmission de données (15, 15', 15) déterminé sur la base du questionnement (100) concernant le signe distinctif pour la modification du protocole de transmission de données (15, 15', 15") de l'interface de transmission de données (35).

17. Procédé pour l'exploitation d'une couche de transmission de données destinée à un appareil médical personnel (20), implantable, programmable, notamment, au moyen d'un agencement (10) selon l'une des revendications 1 à 15, avec les étapes de procédé :
- de questionnement (100) concernant un signe distinctif (30) d'un protocole de transmission de données (15, 15', 15") d'un appareil personnel (20) parmi une multiplicité d'appareils médicaux personnels (20), implantables, programmables, sur la base d'une identification (45) de l'appareil personnel (20), où le protocole de transmission de données (15, 15', 15") fait partie d'un protocole d'encryptage ou de décryptage, ou d'un protocole d'authentification et d'une multiplicité de protocoles de transmission de données (15, 15', 15") qui sont des protocoles d'encryptage et de décryptage, ainsi que des protocoles d'authentification, et où chaque protocole de transmission de données (15, 15', 15") de la multiplicité des protocoles de transmission de données (15, 15', 15") est muni d'un signe distinctif (30), qui est conçu pour différencier le protocole de transmission de données (15, 15', 15") d'au moins un autre protocole de transmission de données (15, 15', 15") parmi la multiplicité de protocoles de transmission de données (15, 15', 15"),
- de mise à disposition (105) d'au moins un protocole de transmission de données (15, 15', 15") parmi une multiplicité de protocoles de transmission de données (15, 15', 15") qui est compatible avec le protocole de transmission de données (15, 15', 15") de l'appareil personnel (20) ou est prévu pour la modification d'un protocole de transmission de données (15, 15', 15") d'un appareil personnel (20),
**caractérisé en ce que** le procédé présente en outre en tant qu'étapes de procédé :
- la mise à disposition (115) du protocole de transmission de données (15, 15', 15") compatible avec le protocole de transmission de données (15, 15', 15") de l'appareil personnel (20) sur la base du questionnement (100) concernant le signe distinctif pour un appareil externe (55)
et/ou
- la transmission (110) d'un protocole de transmission de données (15, 15', 15") déterminé sur la base du questionnement (100) concernant le signe distinctif à une unité de commande (40) de l'appareil personnel (20) pour la modification du protocole de transmission de données (15, 15', 15") de l'interface de transmission de données (35).
